# EUROPEAN PATENT APPLICATION

(11) **EP 3 772 650 A1**
(43) Date of publication of application: **10.02.2021**
(21) Application number: 19190771.6
(22) Date of filing: 08.08.2019
(51) Int. Cl.: G01N 33/18, G01N 27/48, B01L 3/00, G01N 27/49

(54) **AN AUTOMATED HEAVY METAL SENSOR**

(71) Applicant: Robert Bosch GmbH, 70442 Stuttgart (DE)
(72) Inventor: GAN, Bee Keen, Singapore, 598731 (SG); ZHANG, Chi, Singapore, 570266 (SG)

(57) **Abstract**

There is provided an automated heavy metal sensor for quantifying at least one heavy metal in water, comprising an inlet (3) and an outlet (8) for water, and a connected fluidic pathway between the inlet (3) and the outlet (8);the connected fluidic pathway comprising a first fluidic pathway (14), a second fluidic pathway (9) and a united pathway (15), the first fluidic pathway (14) starting from the inlet (3) and being united with a second fluidic pathway (9), the second fluidic pathway (9) having as its source an acid array chamber (4), thereby providing the united fluidic pathway (15), the united fluidic pathway (15) passing a mixing region (5), and subsequently a testing chamber (6) comprising at least one working electrode and one auxiliary electrode (7), and finishing in the outlet (8); and wherein the acid array chamber (4) comprises a plurality of containers (13) comprising an acidifying agent. There is also provided a water filtration system and a plumbing system comprising the heavy metal sensor as described herein, as well as a method of using the heavy metal sensor.

## Description

### Technical Field

The disclosure relates to an automated heavy metal sensor for quantifying at least one heavy metal in water by using anodic stripping voltammetry. The disclosure also relates to the automated heavy metal sensor being integrated in a water purification system or in a plumbing system and to a method of quantifying at least one heavy metal in water.

### Background

The detection and quantification of heavy metal ions is important in many applications, including environmental monitoring, waste management, developmental biology, and clinical toxicology. Water and soil contamination with heavy metals is a critical global issue because it is a threat to general health.

There are a handful of technologies that can be applied for the quantitative detection of heavy metal ions in water, such as atomic absorption (AA) spectrometry, inductively coupled plasma atomic emission (ICP-AE), mass spectroscopy (MS), X-ray fluorescence (XRF), colorimetry, and ion-selective electrode (ISE). They are suitable for trace metal analysis in a laboratory setting. However, they often require well controlled experimental conditions, expensive instrumentation, and frequent maintenance and calibration. For these reasons, the methods mentioned above are not particularly well-suited for rapid analysis.

A heavy metal sensor as an addition to water filtration systems or plumbing systems to be used as a home appliance for immediate quantification would therefore be desirable.

Hence, there is a need for the development of an automated heavy metal sensor, eliminating, or at least ameliorating, the problems as discussed above.

### Summary

In a first aspect, there is provided an automated heavy metal sensor for quantifying at least one heavy metal in water. The automated heavy metal sensor may include an inlet and an outlet for water. The automated heavy metal sensor may further include a connected fluidic pathway between the inlet and the outlet. The connected fluidic pathway may include a first fluidic pathway, a second fluidic pathway and a united pathway. The first fluidic pathway starts from the inlet and is united with a second fluidic pathway, the second fluidic pathway having as its source an acid array chamber, thereby providing the united fluidic pathway. The united fluidic pathway passes a mixing region, and subsequently a testing chamber including at least one working electrode and one auxiliary electrode, and finishes in the outlet. The acid array chamber may include a plurality of containers including an acidifying agent.

In a second aspect, there is provided a water filtration system including an integrated automated heavy metal sensor as defined herein.

In a third aspect, there is provided a plumbing system including an integrated automated heavy metal sensor as defined herein.

In a fourth aspect, there is provided a method of quantifying at least one heavy metal in water using an automated heavy metal sensor. The automated heavy metal sensor may include an inlet and an outlet for water, and a fluidic pathway connected between the inlet and the outlet. The connected fluidic pathway may include a first fluidic pathway, a second fluidic pathway and a united fluidic pathway. The method may include a first step of introducing the water through the inlet to the first fluidic pathway of the automated heavy metal sensor. The method may further include a second step of directing water from the first fluidic pathway to be united with an acidifying agent from the second fluidic pathway thereby providing the united fluidic pathway. The method may further include a third step of directing the water together with the acidifying agent in the united fluidic pathway into a mixing region to obtain pretreated water. The method may further include a fourth step of directing the pretreated water to a testing chamber including at least one working electrode and one auxiliary electrode, and quantifying the at least one heavy metal in the pretreated water. The method may further include a fifth step of draining the pretreated water through the outlet. The acid array chamber may include a plurality of containers including the acidifying agent.

### Brief description of drawing

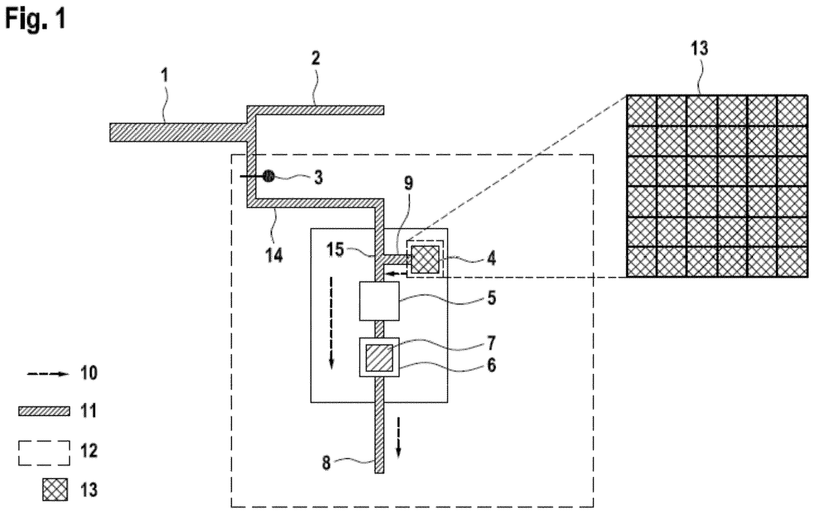

FIG. 1 is a graphic illustration of the fluidic mechanism for online quantification of heavy metal concentration from water. The graphic illustration shows a water pipe 1 which is designated to open out into a tap 2. By branching the water flow 11, the flow of the water may be directed to the first fluidic pathway 14 of the automated system 12. The first fluidic pathway 14 may enter the automated system 12 from the home water pipe 1 via an inlet 3. Following the flow direction 10, the first fluidic pathway 14 is united with a second fluidic pathway 9, having as its source an acid array chamber 4, thereby providing the united fluidic pathway 15. The united fluidic pathway 15 may pass a mixing region 5, and subsequently a testing chamber 6. The testing chamber 6 may include at least one working electrode 7, one auxiliary electrode and optionally one reference electrode. The united fluidic pathway may finish in an outlet 8. The acid array chamber 4 may include a plurality of containers 13 including an acidifying agent. When in measurement, the inlet 3, which may be a valve, is switched to the first fluidic pathway 14 and the water gets acidified in the mixing region 5, followed by heavy metal quantification in the testing chamber 6. Each acid container 13 on the array is intended for one use and the entire automated heavy metal sensor may be replaced when all the acid containers 13 are emptied.

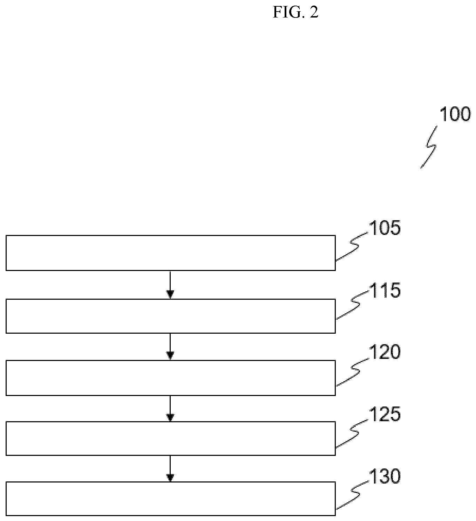

FIG. 2 is a process flow chart describing the individual method steps of the method of quantifying at least one heavy metal in water using an automated heavy metal sensor 100. The method 100 uses an automated heavy metal sensor including an inlet and an outlet for water, and a connected fluidic pathway between the inlet and the outlet; the connected fluidic pathway including a first fluidic pathway, a second fluidic pathway and a united fluidic pathway. The method steps are defined as follows: In step 105, water is introduced through the inlet to the first fluidic pathway of the automated heavy metal sensor. In step 110, the water is directed from the first fluidic pathway to be united with an acidifying agent from the second fluidic pathway thereby providing the united fluidic pathway. In step 115, the water is directed together with the acidifying agent in the united fluidic pathway into a mixing region to obtain pretreated water. In step 120, the pretreated water is directed to a testing chamber including at least one working electrode, one auxiliary electrode and optionally one reference electrode, and the at least one heavy metal is quantified in the pretreated water. In step 125, the pretreated water is drained through the outlet. The acid array chamber includes a plurality of containers including the acidifying agent.

### Detailed Description

Herein, an automated heavy metal sensor is proposed. The automated heavy metal sensor may be, for example, a built-in sample acidification and testing sensor using microfluidics, or an integrated chip. With this automated heavy metal sensor, it is possible to acidify the sample automatically in small volumes of water channeled into the automated system, i.e. the microfluidic reservoir, making online heavy metal quantification possible. Accordingly, in a first aspect, there is provided an automated heavy metal sensor for quantifying at least one heavy metal in water. The automated heavy metal sensor may include an inlet and an outlet for water. The automated heavy metal sensor may further include a connected fluidic pathway between the inlet and the outlet. The connected fluidic pathway may include a first fluidic pathway, a second fluidic pathway and a united fluidic pathway. The first fluidic pathway may start from the inlet and may be united with a second fluidic pathway. The second fluidic pathway may have as its source an acid array chamber. Uniting the first fluidic pathway and the second fluidic pathway may provide the united fluidic pathway. The united fluidic pathway may pass a mixing region. Subsequently, the united fluidic pathway may pass a testing chamber. The testing chamber may include at least one working electrode, one auxiliary electrode and optionally one reference electrode. The united fluidic pathway may finish in the outlet. The acid array chamber may include a plurality of containers including an acidifying agent.

The automated heavy metal sensor according to the first aspect provides for quantifying heavy metals in an "online mode". This is because all steps in relation with sample preparation and quantification may be implemented within one connected fluidic pathway, including the first fluidic pathway, the second fluidic pathway and the united fluidic pathway. Accordingly, the automated heavy metal sensor may be built as a testing chip, wherein acidification of the water is followed directly by quantification within the same micro fluidic system. Furthermore, due to the use of microfluidic technology in the connected fluidic pathway, the automated heavy metal sensor may be sufficiently small to be used in a domestic setting, for example as a home appliance.

The connected fluidic pathway including the first fluidic pathway, the second fluidic pathway and the united fluidic pathway may be formed by channels, preferably microchannels, to guide water or the acidifying agent there through. The connected fluidic pathway may also be formed by pipes or tunnels wherein water or the acidifying agent may pass through. The connected fluidic pathway may also provide an essentially "closed system" for the water or the acidifying agent to be confined within the automated heavy metal sensor, for example, with the only connection to the exterior of the automated heavy metal sensor being the inlet and the outlet. The connected fluidic pathway may have a flow direction for guiding water or an acidifying agent there through, starting at the inlet and finishing in the outlet. Thus, the sensor may be configured so that the water stream flows in a flow direction 10 from the inlet to the outlet. The flow direction may be generated by an incoming water pressure. For example, as the inlet may be connectable to a home water pipe, the water pressure being on this home water pipe may result in the flow direction in the automated heavy metal sensor. Additionally, capillary forces may result in the water sample being drawn into the flow direction.

The above automated heavy metal sensor may be configured to be utilized in Anodic Stripping Voltammetry (ASV). ASV is an analytical technique, wherein it is possible to quantify heavy metals with a very low quantification limit, up the nanomolar region, while being very selective for the heavy metal being analyzed. The underlying principle involves the heavy metal of interest being electroplated on the at least one working electrode during a deposition step, and oxidized from the at least one working electrode during the stripping step. The current is measured during the stripping step. The oxidation of the heavy metal is registered as a peak in the current signal at the potential at which the heavy metal begins to be oxidized. The stripping step can be, for example, linear, staircase, squarewave, or pulse.

The heavy metals which are quantified by the automated heavy metal sensor may include, for example, lead, iron, copper, arsenic, nickel, manganese, mercury, zinc, cadmium, chromium, and a combination thereof. In some embodiments, the heavy metals which are quantified by the automated heavy metal sensor may include, for example, lead, arsenic, cadmium, chromium, mercury or a combination thereof. It is understood that the term "heavy metal" includes heavy metals in their elemental state, as well as in their ionized state.

The water may originate from a home water pipe, designated to flow into a home water tap. Accordingly, the water to be used for the quantification may be a tap water sample. Meanwhile, the water that is provided to the automated heavy metal sensor may be branched off the water pipe by a suitable fitting, for example a diverter tee. By branching the water flow, the flow of the water may be directed into the automated heavy metal sensor.

In some embodiments, the inlet may include means for selectively permitting or preventing flow of water there through.

In some embodiments, the means for selectively permitting or preventing flow of the water may include at least one valve. In some embodiments, the at least one valve may comprise at least one pneumatic valve. Because of the use of microfluidic technology, it is possible to use very small quantities of the water for the quantification. Accordingly, in some embodiments, the at least one valve (for example, the at least one pneumatic valve) may be configured to permit less than 20 mL, or less than 15 ml, or between about 5 ml to about 10 mL of the water to flow through per each quantification of the at least one heavy metal in water. Such small quantities facilitate use of the automated heavy metal sensor as a home appliance, as it does not require a lot of space. In fact, the entire automated heavy metal sensor, including mixing region and testing chamber, may be built-in on a testing chip.

The water being analyzed may undergo a pretreatment, involving addition of an acidifying agent. This pretreatment may be provided to reduce interference with other metals or impurities. In particular, acidification of the water as part of the pretreatment may be beneficial in order to ensure stable ion exchange process during quantification. In the automated heavy metal sensor as disclosed herein, this pretreatment may be carried out automatically by providing the acidifying agent from the second fluidic pathway. Accordingly, the acidifying agent may be configured to acidify the water. Pretreated water may thereby be obtained. By using the automated heavy metal sensor as described herein for the pretreatment, lengthy sample preparation, which is commonly encountered in ASV especially when other interfering metals or impurities are present in the water, can be avoided. In particular, by carrying out the pretreatment automatically, the time for carrying out the pretreatment may be reduced to less than about 10 minutes, or to less than about 1 minute. In some embodiments, the time for carrying out the pretreatment may be reduced to a range of about 1 minute to about 10 minutes, or to a range of about 1 minute to about 5 minutes.

In the disclosed automated heavy metal sensor, the acidifying agent to be added may be stored in an acid array chamber. In one embodiment, the acid array chamber may include more than 10 containers, or more than 20 containers, or more than 30 containers. Each of the containers may be configured to release equal quantities of the acidifying agent. The quantity of the acidifying agent to be released during measurement may be configured to obtain a desired pH value in the pretreated water, dependent on the quantity of the water to be measured and on the pH value of the acidifying agent. Replacement of the automated heavy metal sensor may be necessary once all of the containers have released the acidifying agent contained therein. The quantity of the acidifying agent to be released during measurement may be dependent on the amount of water to be tested. Alternatively, or additionally, the quantity of the acidifying agent to be released during measurement may be dependent on the molarity of the acidifying agent. In one example, 5 mL of water may be used and acidified with 2 mL of a 1-molar solution of acidifying agent. Alternatively, 5 mL of water may be used and acidified with 1 mL of a 2-molar solution of acidifying agent.

Each of the containers may be selected for each measurement by a mechanical control. Alternatively, each of the containers may be selected for each measurement by a digital control. For each quantification of at least one heavy metal, the selection of the container may be triggered by intention. Hence, the selection of the container may be triggered by the user. Alternatively, the selection of the container may be triggered by the system. In one example, a mechanical movement of the acid array chamber, containing the plurality of containers, may place a selected container for each measurement into a physical position to be in connection with the second fluidic pathway. This may facilitate multiple quantifications across time.

In one embodiment, the acidifying agent may be configured to adjust the pH of the water to a pH value of 2 to 6, optionally to a pH value of 3 to 4. The pH value range of 3 to 4 may help to protect the at least one working electrode while performing ASV.

In one embodiment, the acidifying agent may be an acid. Acids which may be suitable for being used in the container of the automated heavy metal sensor include nitric acid, hydrofluoric acid, sulphuric acid, hydrochloric acid, or a combination thereof. In one example, the acid may be hydrochloric acid.

Alternatively, the acidifying agent may be a buffer. A buffer may be a compound or a mixture of compounds that, when present in a solution, may resist changes in the pH of the solution when small quantities of acid or base are added to the solution. Buffers which may be suitable for being used in the container of the automated heavy metal sensor include phosphate buffer, acetate buffer, sulfonic acid buffer, ethanoate buffer, or a combination thereof. In one example, the buffer may be a sulfonic acid buffer, such as HEPES (4-(2-hydroxyethyl)-1-piperazineethanesulfonic acid) buffer.

During operation of the automated heavy metal sensor, one of the containers including the acidifying agent may be opened to release the acidifying agent to the second fluidic pathway. As the first fluidic pathway is in fluidic connection with the second fluidic pathway, when in use, the water is united with the released acidifying agent.

Subsequent to the first fluidic pathway being united with the second fluidic pathway, the united fluidic pathway passes a mixing region. In other words, the mixing region may be located downstream of the first fluidic pathway and the second fluidic pathway. The mixing region may be a space designated to mix the water with the acidifying agent. The mixing region may interpose the united fluidic pathway.

In one embodiment, the mixing region may be a mixing channel, for example, the mixing channel may have the same channel boundaries as the united fluidic pathway. The mixing may thereby be accompanied or facilitated by the water flow. Hence, mixing in the mixing channel may be referred to as "in-line-mixing".

Alternatively, the mixing region may be a mixing chamber. In this embodiment, the mixing chamber may be a separate compartment from the united fluidic pathway. Hence, the water flow may pass the mixing chamber, wherein the water may be mixed with the acidifying agent, before continuing in flow direction. The mixing chamber may facilitate mixing the water with the acidifying agent from the united fluidic pathway. The mixing may result in the water to be pretreated.

The united fluidic pathway continues in the flow direction to the testing chamber. The quantification using ASV may involve several steps. The pretreated water may be stirred during the first two steps at a repeatable rate. The first step may be a cleaning step; in the cleaning step, the potential may be held at a more oxidizing potential than the at least one heavy metal to be quantified for a period of time in order to fully remove it from the at least one working electrode. In the second step, the potential may be held at a lower potential, low enough to reduce the at least one heavy metal and deposit it with accumulation on the surface of the working electrode. After the second step, the stirring may be stopped, and the working electrode may be kept at the lower potential. The third step allows the deposited material to be distributed more evenly on the working electrode. The next step may involve raising the working electrode to a higher potential (anodic), and stripping (oxidizing) the at least one heavy metal. As the at least one heavy metal is oxidized, it gives off electrons which may be measured as a current with respect to voltage, for example sweeping voltages, and/or scanning time.

The quantification may involve at least two electrodes, for example at least one working electrode and at least one auxiliary electrode. In some embodiments, the quantification may involve at least three electrodes, including at least one working electrode, at least one auxiliary electrode, and at least one reference electrode. The at least one working electrode may be a platinum electrode, a gold electrode, a silver electrode or a carbon electrode. Such electrode may be in a disk or planar strip configuration. The at least one auxiliary electrode may be a platinum electrode. In case a reference electrode is used, this electrode may be a silver/silver chloride electrode.

In some embodiments, the at least one working electrode, one auxiliary electrode and optionally one reference electrode may be connected to a quantification circuit for detection and/or quantification of the at least one heavy metal. The quantification circuit may allow for a result, i.e. the quantification of the heavy metal, to be displayed to a user. The result may be sent to the user via wireless communication. The wireless communication may involve, for example, cloud-storage, wifi, Bluetooth, etc. The user may then be informed of the water quality, for example of the tap water quality in his home.

The water, after quantification of the at least one heavy metal therein, may exit the connected fluidic pathway through an outlet. The outlet may be a drain.

The automated heavy metal sensor may be integrated into a water filtration system. Accordingly, in a second aspect, there is provided a water filtration system including an integrated automated heavy metal sensor as defined above. This water filtration system may be realized as a home appliance, due to the small size of the automated heavy metal sensor. Based on the results of the quantification, the user may make an informed decision as the when to replace/fix the water filtration system at home.

The automated heavy metal sensor may be integrated into a plumbing system. Accordingly, in a third aspect, there is provided a plumbing system including an integrated automated heavy metal sensor as defined above. This plumbing system may be realized as a home appliance, due to the small size of the automated heavy metal sensor.

In a fourth aspect, there is provided a method of quantifying at least one heavy metal in water using an automated heavy metal sensor. The automated heavy metal sensor may include an inlet and an outlet for water. The automated heavy metal sensor may further include a connected fluidic pathway between the inlet and the outlet. The connected fluidic pathway may include a first fluidic pathway, a second fluidic pathway and a united fluidic pathway. The method may include a first step of introducing the water through the inlet to the first fluidic pathway of the automated heavy metal sensor. The method may further include a second step of directing water from the first fluidic pathway to be united with an acidifying agent from the second fluidic pathway thereby providing the united fluidic pathway. The method may further include a third step of directing the water together with the acidifying agent in the united fluidic pathway into a mixing region to obtain pretreated water. The method may further include a fourth step of directing the pretreated water to a testing chamber including at least one working electrode and one auxiliary electrode, and quantifying the at least one heavy metal in the pretreated water. The method may include a fifth step of draining the pretreated water through the outlet. The acid array chamber may include a plurality of containers including the acidifying agent. The first, second, third, fourth and fifth step may be in chronological sequence. In case one of the steps should be omitted, it is clear that the numbering of the steps may then be adapted to a lower number than five steps.

The method of the fourth aspect may be carried out by using the automated heavy metal sensor of the first aspect. Further, the method may be carried out automatically. Hence, the method may be carried out once a corresponding signal is received from the user upon which the entire method may be carried out automatically, including acidification and quantification.

In one embodiment of the method, the at least one working electrode may be regenerated by applying a negative potential. The negative potential may in the range of -0. IV to about -0.5V, optionally in the range of -0.2V to about -0.4V, optionally in the range of -0.3V to about -0.4V, when measured against the reference electrode. The regeneration time may be between about 1 second to about 120 seconds, or about 5 seconds to about 50 seconds, or about 10 seconds to about 30 seconds.

### Example

As depicted in FIG. 1, the disclosure relates to an automated heavy metal sensor, which may be a fluidic chip that can be integrated into the plumbing system to quantify heavy metals in tap water.

In the case when measurements are needed, the valve to the fluidic chip is opened and water from the pipe 1 will go into the first fluidic pathway 14 for subsequent measurement. 5 to 10 mL of water may be used for testing and the volume can be controlled by built-in pneumatic valves of the inlet 3. As shown by the arrow signifying the second fluidic pathway 9, the pre-stored acidifying agent, such as buffer or acid, comes from one of the many containers 13 in the array 4. The acidifying agent unites with the first fluidic pathway 14 to provide united fluidic pathway 15 and gets mixed with water (the sample to be tested) in the mixing region 5. Hence, the sample is acidified upon thorough mixing and subsequently flows in the flow direction 10 into the testing chamber 6 where electrodes 7 are positioned and the quantification of the heavy metal can be realized. When the measurement is done, the electrode 7 can be regenerated by applying a negative potential at -0.3V to -0.4V when measured against the reference electrode for about 10 to 30 seconds while still being immersed in the tested samples and thereafter, the tested sample is emptied from the chambers to the outlet 8, which may be a drain, and the valve to the fluidic chip is closed. For subsequent measurements, acidifying agent from the next container may be used and the same procedure repeats.

When all the acidifying agent in the container array 4 is used and emptied, the fluidic chip may be replaced.

The results of the measurements can be transferred via wifi or cloud-storage to the user to provide the information regarding their water quality. The operation of the fluidic chip may thus be automated and the users will only have to switch on the measurement mode.

In the case when measurements are not needed, the valve to the fluidic mechanism is closed and water can flow from the pipe 1 to the tap 2.

## Claims

1. An automated heavy metal sensor for quantifying at least one heavy metal in water, comprising an inlet (3) and an outlet (8) for water, and a connected fluidic pathway between the inlet (3) and the outlet (8);
the connected fluidic pathway comprising a first fluidic pathway (14), a second fluidic pathway (9) and a united pathway (15), the first fluidic pathway (14) starting from the inlet (3) and being united with a second fluidic pathway (9), the second fluidic pathway (9) having as its source an acid array chamber (4), thereby providing the united fluidic pathway (15), the united fluidic pathway (15) passing a mixing region (5), and subsequently a testing chamber (6) comprising at least one working electrode and one auxiliary electrode (7), and finishing in the outlet (8); and
wherein the acid array chamber (4) comprises a plurality of containers (13) comprising an acidifying agent.

2. The automated heavy metal sensor of claim 1, wherein the inlet (3) further comprises at least one pneumatic valve.

3. The automated heavy metal sensor of claim 2, wherein the at least one pneumatic valve is configured to permit less than 20 mL of water to flow through per quantification of the at least one heavy metal in water.

4. The automated heavy metal sensor of any one of the preceding claims, wherein the acidifying agent is configured to adjust the pH of water to a value of pH 3 to 4.

5. The automated heavy metal sensor of any one of the preceding claims, wherein the acidifying agent is an acid.

6. The automated heavy metal sensor of any of the preceding claims 1 to 4, wherein the acidifying agent is a buffer.

7. The automated heavy metal sensor of any one of the preceding claims, wherein the mixing region is a mixing channel.

8. The automated heavy metal sensor of any of the preceding claims 1 to 6, wherein the mixing region is a mixing chamber.

9. The automated heavy metal sensor of any one of the preceding claims, wherein the at least one working electrode and one auxiliary electrode is connected to a quantification circuit for detection and/or quantification of the at least one heavy metal.

10. A water filtration system comprising an integrated automated heavy metal sensor of any one of the preceding claims.

11. A plumbing system comprising an integrated automated heavy metal sensor of any one of claims 1 to 9.

12. A method (100) of quantifying at least one heavy metal in water using an automated heavy metal sensor comprising an inlet and an outlet for water, and a connected fluidic pathway between the inlet and the outlet; the connected fluidic pathway comprising a first fluidic pathway, a second fluidic pathway and a united fluidic pathway, the method comprising:
i) introducing the water through the inlet to the first fluidic pathway of the automated heavy metal sensor (105);
ii) directing water from the first fluidic pathway to be united with an acidifying agent from the second fluidic pathway thereby providing the united fluidic pathway (110);
iii) directing the water together with the acidifying agent in the united fluidic pathway into a mixing region to obtain pretreated water (115);
iv) directing the pretreated water to a testing chamber comprising at least one working electrode and one auxiliary electrode, and quantifying the at least one heavy metal in the pretreated water (120); and
v) draining the pretreated water through the outlet (125);
wherein the acid array chamber comprises a plurality of containers comprising the acidifying agent.
